# EUROPÄISCHE PATENTSCHRIFT

(11) **EP 2 823 856 B1**
(45) Veröffentlichungstag und Bekanntmachung des Hinweises auf die Patenterteilung: **03.02.2016**
(21) Anmeldenummer: 14172272.8
(22) Anmeldetag: 13.06.2014
(51) Int. Cl.: A61N 1/36

(54) **Federkontaktbauteil, Steckerkontaktbuchse und Kontaktbuchsenbauteil**
Spring contact component, plug contact socket, and contact socket component
Composant de contact à ressort, douille de prise et composant de prise de contact

(30) Priorität: 11.07.2013 US 201361844862 P
(43) Veröffentlichungstag der Anmeldung: 14.01.2015
(73) Patentinhaber: BIOTRONIK SE & Co. KG, 12359 Berlin (DE)
(72) Erfinder: Rebentisch, Ronald, 10967 Berlin (DE); Lehmann, Stefan, 16269 Wriezen (DE)
(74) Vertreter: Galander, Marcus

(56) Entgegenhaltungen:
- JP-A- 2002 217 046
- JP-A- 2010 147 199
- JP-A- 2011 023 561

## Beschreibung

Die vorliegende Erfindung betrifft ein Federkontaktbauteil für eine Steckerkontaktbuchse eines elektromedizinischen Implantats mit einer den äußeren Umfang einer Steckeröffnung umgebenden Wendel mit einem ersten und einem zweiten (Wendel-)Ende sowie ein Verfahren zu deren Herstellung, ein entsprechendes Kontaktbuchsenbauteil, eine entsprechende Steckerkontaktbuchse sowie ein entsprechendes elektromedizinisches Implantat.

Medizinische Implantate sind in großer Vielfalt aus dem Stand der Technik bekannt. Als elektromedizinisches Implantat wird im Zusammenhang mit der vorliegenden Erfindung ein aktives Implantat verstanden, das neben einer Stromversorgung (z. B. Batterie) weitere elektrische und/oder elektronische Bauteile umfasst (z. B. Kondensatoren), welche in einem Gehäuse angeordnet sind, das hermetisch dicht abgeschlossen ist. Derartige elektromedizinische Implantate sind beispielsweise Herzschrittmacher, Defibrillatoren oder Neurostimulatoren. Diese Implantate dienen dazu, das biologische Gewebe mittels elektrischer Pulse anzuregen, welche von einem in dem Gehäuse vorgesehenen Impulsgenerator erzeugt werden.

Häufig sind derartige Implantate mit Elektrodenleitungen verbunden, welche nach der Implantation in einen Körper diesen therapieren, beispielsweise Stimulationsimpulse und/oder Defibrillatorschocks an bestimmte Körperstellen übertragen und/oder abgeben, oder dazu dienen, elektrische Potentiale von Körperstellen zu erfassen. Hierfür muss eine elektrische Verbindung zwischen den im Gehäuseinneren angeordneten elektrischen und/oder elektronischen Bauteilen und der Elektrodenleitung hergestellt werden. Diese elektrische Verbindung wird in der Regel mittels einer Durchführung und einem sogenannten Anschlussgehäuse realisiert. Eine Durchführung sorgt dabei für mindestens eine elektrische Verbindung zwischen dem Inneren des Gehäuses und dem Äußeren und ist gleichzeitig für den hermetischen Abschluss des Gehäuses verantwortlich. Das über der Durchführung befestigte Anschlussgehäuse führt die elektrische Verbindung der Durchführung weiter zu einer Kontaktstelle, die häufig als Steckerkontaktbuchse gestaltet ist. In die meist genormte Steckerkontaktbuchse wird nach der Implantation das Anschlussstück (Stecker) der Elektrodenleitung eingeführt. Hierdurch wird an den Kontaktstellen der Steckerkontaktbuchse ein elektrischer Kontakt zwischen den elektronischen Bauteilen des Implantats, z. B. einem Impulsgenerator, und den Kontaktstellen des Anschlussstücks der Elektrodenleitung und somit der an dieser befestigten Elektrode hergestellt.

Die Schnittstelle zwischen dem Implantat und der Elektrodenleitung ist üblicherweise als lösbare Mehrfachsteckverbindung ausgelegt und wird in ihrer Geometrie über ISO-Normen definiert. In der Norm ISO 5841-3 (IS-1-Norm) wird für Impulsgeneratoren mit Herzschrittmacherfunktion, d. h. für Impulsgeneratoren, die mit Niederspannungspulsen arbeiten, eine koaxial-gestufte, zweifache Steckverbindung mit Dichtlippen festgelegt. In der Norm ISO 27186 (DF4/IS4-Norm) wird für Impulsgeneratoren mit Herzschrittmacherund/oder Defibrillatorfunktion, d. h. mit Hoch- und Niederspannungspulsen, eine koaxial-gestufte, vierfache Steckverbindung (davon drei in-line und glatt) mit in der Buchsenöffnung integrierten Dichtlippensystemen festgelegt. Demgegenüber existieren keine Normvorgaben für eine Stecker-/Buchsengeometrie von Neurostimulatoren. Bei zum Zeitpunkt der Einreichung vorliegender Anmeldung bekannten Produkten werden z. B. Stecker-/Buchsensysteme mit koaxial-glatten achtfachen Steckverbindungen mit in die Buchsenöffnung integrierten Dichtlippensystemen verwendet.

Bei den genannten Stecksystemen können grundsätzlich alle Kontaktierungen über Federkontakte realisiert werden, so lange bestimmte Anforderungen hinsichtlich der Einsteck-, der Rückhalte- und der Auszugskraft sowie der elektrischen Kontaktstabilität eingehalten werden.

Bei den bekannten Steckerkontaktbuchsen werden die Dichtlippensysteme bei lebenserhaltenden Implantaten (Herzschrittmacher, Defibrillatoren) üblicherweise redundant ausgelegt, d. h. es kommen beidseitig der einzelnen elektrischen Kontaktbereiche Dichtlippenpaare zum Einsatz, um einerseits Körperflüssigkeit von der Kontaktstelle fernzuhalten und andererseits die Kontaktstellen elektrisch voneinander zu isolieren. Bei nicht lebenserhaltenden Implantaten (z. B. Neurostimulatoren) wird von dieser Regel auch abgewichen.

Aus der Druckschrift WO 2006/026186 A2 ist ein Implantat mit einer Steckerkontaktbuchse für eine Mehrfach-Steckverbindung bekannt, welche mehrere elektrisch leitende, torusförmige Kontaktfedern (Federwendeln) aufweist. Dabei ist jede Federwendel in einem entsprechenden, metallischen Federring derart angeordnet, dass die Federwendel den äußeren Umfang der Öffnung für die Einführung des Steckers an der jeweiligen Stelle umgibt. Ferner ist an jedem Federring eine an diesem befestigte Drahtzuleitung vorgesehen, welche den elektrischen Kontakt zu dem Inneren des Implantatgehäuses herstellt. Die Einheit aus einer Federwendel, dem zugehörigen Federring und der an diesem befestigte Drahtzuleitung wird auch als Federkontaktbauteil bezeichnet. Zusammen mit dem ebenfalls im obigen Dokument offenbarten Kunststoffgehäuse ist ein Kontaktbuchsenbauteil gebildet.

Bei weiteren bekannten Lösungen wird auf den Federring verzichtet und die torusförmige Kontaktfeder wird in einem Federkäfig aus elektrisch leitendem Material gelagert. Der Federkäfig ist dabei häufig als ein metallisches Formfrästeil hergestellt. Das Kontaktbuchsenbauteil wird manuell aus Federwendel und Federkäfig zusammengesetzt. In einem weiteren manuellen Verfahrensschritt werden Steckerkontaktbuchsen durch abwechselndes Aneinanderreihen von Kontaktbuchsenbauteilen und Dichtbuchsenbauteilen hergestellt. Eine automatisierte Herstellung von derartigen Steckerkontaktbuchsen ist bauartbedingt nicht möglich. Die bekannte Lösung ist zudem bei Berücksichtigung der prozessbedingten Form- und Lagetoleranz ihrer Einzelkomponenten überbestimmt, so dass auf einen in eine solche Steckerkontaktbuchse gesteckten, realen Elektrodenstecker nicht einheitliche Kräfte wirken können, die in der Folge zu Fehlfunktionen führen könnten. Als nachteilig bei einer solchen bekannten Lösung hat sich ferner erwiesen, dass der eingenommene Bauraum vergleichsweise voluminös ist und keine weitere Volumenreduzierung zulässt. Problematisch erscheint zudem die Abdichtung zwischen den modular aufgebauten metallisch leitfähigen Kontaktbuchsenbauteilen und den dazwischen liegenden elektrisch isolierenden Dichtbuchsenbauteilen.

Ein Federkontaktbauteil nach dem einleitenden Teil von Anspruch 1 is aus JP2011023561 bekannt.

Die Aufgabe besteht somit darin, eine Steckerkontaktbuchse für ein Anschlussgehäuse zu schaffen, das obige Nachteile vermeidet und insbesondere auf einfache Weise maschinell und damit kostengünstig herstellbar ist. Die Steckerkontaktbuchse soll dabei auch die oben angegebenen Normen erfüllen.

Die obige Aufgabe wird durch eine Steckerkontaktbuchse gemäß Anspruch 7 mit einem neuartigen Federkontaktbauteil mit den Merkmalen des Anspruchs gelöst.

Erfindungsgemäß sind das zweite Ende (5.2) der Wendel (5) und das draht- und/oder bandförmige Element (10) sowie ein Anschlussstift (12) einstückig ausgebildet.

Alternativ ist ebenfalls erfindungsgemäß mindestens das zweite Ende der die Steckeröffnung umgebenden Wendel des Federkontaktbauteils elektrisch und mechanisch über ein draht- und/oder bandförmiges Element direkt mit einem Anschlussstift verbunden. Der Anschlussstift dient dabei zur elektrischen Verbindung des Federkontaktbauteils mit den in dem Gehäuseinneren des Implantats angeordneten elektrischen/elektronischen Bauteilen über den Header und die Durchführung des Implantats. Bei beiden alternativen Lösungen kann das Gehäuse des Kontaktbuchsenbauteils aus demselben Werkstoff oder derselben Werkstoffgruppe (beispielsweise nicht leitende Polymere) bestehen, aus dem auch die Dichtbuchsenbauteile bestehen. Damit kann auch das Gehäuse des Kontaktbuchsenbauteils aus einem elektrisch isolierenden Werkstoff in Form eines kostengünstigen Spritzgussteils gefertigt werden. Der elektrisch isolierende Werkstoff kann so gewählt werden, dass die unterschiedlichen Buchsenbauteile miteinander verklebt werden können. So wird vorteilhafterweise ein mögliches Eindringen von Flüssigkeit in die elektrischen Kontaktbereich verhindert.

Die Definition, dass ein draht- und/oder bandförmiges Element die direkte Verbindung mit dem Anschlussstift darstellt, beinhaltet, dass weitere voluminöse Elemente zwischen dem Anschlussstift und der Wendel nicht vorhanden sind.

In einem bevorzugten Ausführungsbeispiel bildet das erste Ende der Wendel einen Anschlussstift aus.

Der Verdienst der Erfinder besteht darin, einen einfacheren Aufbau des Federkontaktbauteils gefunden zu haben, bei dem der Anschlussstift in die Wendel integriert ist oder der Anschlussstift lediglich über ein draht- und/oder bandförmiges Element mit der Wendel elektrisch oder mechanisch verbunden ist. Es kann also auf weitere elektrische Zuleitungsbauteile verzichtet werden kann, was die Herstellung weiter vereinfacht. Der einfachere Aufbau des Federkontaktbauteils führt zu geringeren Kosten bei der Herstellung, welche nun automatisiert werden kann. Mit den erfindungsgemäßen Federkontaktbauteilen können modulare Steckerbuchsen gefertigt werden, welche den oben angegebenen Normen entsprechen.

In einem bevorzugten Ausführungsbeispiel weist das draht- und/oder bandförmige Element ein die Wendel im Bereich ihres Umfangs außen umgebendes formstabilisierendes Mittel auf. Bei diesem bevorzugten Ausführungsbeispiel kann erfindungsgemäß das herkömmliche metallische Gehäuse (Federkäfig), der die Wendel bisher aufgenommen hatte und als Formfrästeil ausgebildet war, entfallen. In einer bevorzugten Ausführungsform der Erfindung ist das formstabilisierende Mittel als einfacher oder mehrfacher Ring oder als Spirale ausgebildet, der vorzugsweise am äußeren Umfang der innerhalb des einfachen oder mehrfachen Rings liegenden Wendel anliegt und diese umgibt. Ein derartiger ein- oder mehrfacher Ring wird gewissermaßen außen im Bereich des Umfangs der Wendel um die Wendel gelegt und bewirkt dadurch eine Formstabilisierung.

Es ist weiter von Vorteil, wenn das formstabilisierende Mittel an mehreren Stellen mit der Wendel verbunden ist, z. B. über Laserschweißpunkte oder durch elektrisch leitfähige, selbstaushärtende Kunststoffe oder Klebstoffe.

Das Federkontaktbauteil ist vorzugsweise aus elektrisch leitendem Material gefertigt, z. B. aus federharten Implantatlegierungen, aus einer Platin-Iridium-Legierung, besonders bevorzugt aus Pt80Ir20. Zu weiterer Kosteneinsparung führt, wenn das draht- und/oder bandförmige Element und die Wendel der Alternativlösung aus dem gleichen, elektrisch leitenden Material gefertigt sind. Die elektrische Spannung, z. B. ein Spannungspuls, wird dann von dem Inneren des Implantatgehäuses über die Durchführung und den Header, den Anschlussstift, das draht- und/oder bandförmige Element und die Wendel zum Stecker geleitet.

In einer bevorzugten Ausführungsform des erfindungsgemäßen Kontaktbuchsenbauteils ist das Federkontaktbauteil in ein Gehäuse aus einem elektrisch nichtleitenden Material, vorzugsweise ein mittels Spritzguss gefertigtes Gehäuse, eingelegt oder eingebettet, wobei der ersten Anschlussstift und der zweite Anschlussstift vorzugsweise im Bereich des äußeren Umfangs des Gehäuses aus dem Gehäuse herausragen. Das Federkontaktbauteil ist dort mit seinem außen umgebenden formstabilisierenden Mittel eingelegt. Ein derartiges Kontaktbuchsenbauteil lässt sich einfach herstellen, wobei das Spritzgussteil vorzugsweise aus einem Elastomer besteht und besonders bevorzugt Silikon enthält. Bei dieser erfindungsgemäßen Lösung wird ein kostenintensives Formfrästeil durch ein kostengünstiges Spritzgussteil ersetzt, das zudem kleinbauender ausgeführt werden kann, da das draht- und/oder bandförmige Element bereits die wesentliche formstabilisierende Funktion übernimmt. Da die metallischen Formfrästeile häufig aus dem Werkstoff MP35N gefertigt sind, wird durch ihren Wegfall zudem das Risiko von Langzeitkontaktinstabilitäten vermindert.

Das Gehäuse kann beispielsweise als Zylinderabschnitt mit mittiger, durchgehender Öffnung gestaltet sein, das im Bereich der innen liegenden Wand der Öffnung eine Kerbe zur Aufnahme des Federkontaktbauteils aufweist. Ferner kann mindestens eine im Wesentlichen radial verlaufende durchgehende Öffnung in dem Gehäuse vorgesehen sein, welche sich in einer besonders bevorzugten Ausführungsform bis in die Kerbe für das Federkontaktbauteil erstreckt. In der radial verlaufenden Öffnung werden beispielsweise das erste und zweite Ende der Wendel, die beide als Anschlussstift ausgebildet sind, angeordnet. Nach dem Einlegen oder Einbetten des Federkontaktbauteils in das Gehäuse, insbesondere in die an der Innenwand vorgesehenen Kerbe, wird die radial verlaufende Öffnung um den/die herausragenden Anschlussstift(e) beispielsweise mit einem Silikonkleber abgedichtet. Hierdurch wird eine verbesserte Abdichtung der elektrisch leitenden Bauteile erreicht, welche auch eine Abdichtung gegenüber der Körperflüssigkeit darstellt. Ferner wird der benötigte Bauraum für das Kontaktbuchsenbauteil auf ein Minimum beschränkt.

Die obige Aufgabe wird ferner durch ein Anschlussgehäuse für ein elektromedizinisches Implantat gelöst, welcher mindestens zwei nebeneinander angeordnete, oben beschriebene Kontaktbuchsenbauteile aufweist. Dieses auch "Header" genannte Anschlussgehäuse kann auch mehrteilig (modular) aufgebaut sein.

Bevorzugt werden die oben beschriebenen, mindestens zwei Kontaktbuchsenbauteile durch ein dazwischen angeordnetes Dichtbuchsenbauteil getrennt, das als elektrisch isolierendes Spritzgussteil, beispielsweise mit Silikon, ausgebildet sein kann. Ein derartiges Dichtbuchsenbauteil ist vorzugsweise als Kreiszylinderabschnitt mit einer zentrischen, durchgehenden Öffnung ausgebildet, wobei im Bereich der innen liegenden Wand der durchgehenden Öffnung mindestens eine Dichtlippe, besonders bevorzugt ein Dichtlippenpaar, angeordnet ist.

Bei beiden alternativen Lösungen kann das Gehäuse des Kontaktbuchsenbauteils aus demselben Werkstoff oder derselben Werkstoffgruppe (beispielsweise nicht leitende Polymere) bestehen, aus dem auch die Dichtbuchsenbauteile bestehen. Damit kann auch das Gehäuse des Kontaktbuchsenmoduls aus einem elektrisch isolierenden Werkstoff in Form eines kostengünstigen Spritzgussteils gefertigt werden. Der elektrisch isolierende Werkstoff kann so gewählt werden, dass die unterschiedlichen Buchsenbauteile miteinander verklebt werden können. So wird vorteilhafterweise ein mögliches Eindringen von Flüssigkeit in die elektrischen Kontaktbereich verhindert.

Die Steckerkontaktbuchse des erfindungsgemäßen Headers kann auch mehr als zwei Kontaktbuchsenbauteile aufweisen. Vorteilhaft ist, wenn benachbarte Kontaktbuchsenbauteile jeweils durch ein dazwischen angeordnetes Dichtbuchsenbauteil getrennt sind und zusätzlich am offenen Ende der Steckerkontaktbuchse ein Dichtbuchsenbauteil zur Abdichtung vorgesehen ist. Durch diesen modularen Aufbau können beliebige Steckerbuchsenkonfigurationen am Header realisiert werden.

Das die oben beschriebene Aufgabenstellung lösende erfindungsgemäße Verfahren zur Herstellung eines Federkontaktbauteils für einen Steckkontaktbuchse eines elektromedizinischen Implantats mit einer den äußeren Umfang einer Steckeröffnung umgebende Wendel beinhaltet die folgenden Schritte:
- Herstellung einer ersten Wendel und einer zweiten Wendel aus einem Draht oder einem Band, wobei die erste Wendel einen kleineren Durchmesser als die zweite Wendel aufweist,
- Ausbildung eines offenen Torus aus der ersten Wendel mit dem kleineren Durchmesser,
- gegebenenfalls Verbinden der ersten Wendel und der zweiten Wendel an jeweils einem Ende jeder Wendel miteinander,
- Eindrehen der ersten Wendel in Form eines offenen Torus in die zweite Wendel derart, dass die zweite Wendel einen die erste Wendel im Bereich ihres Umfangs außen umgebenden einfachen oder mehrfachen Ring ausbildet.

Falls die erste Wendel und die zweite Wendel von Beginn an aus einem einzigen Draht oder Band gefertigt werden, was vorteilhaft ist, entfällt der Verfahrensschritt, in dem die erste Wendel und die zweite Wendel miteinander verbunden werden.

In einem bevorzugten Ausführungsbeispiel des erfindungsgemäßen Verfahrens wird die erste Wendel vor der Ausbildung des Torus geschränkt, wodurch die Federeigenschaften der ersten Wendel beeinflusst werden.

Um eine bessere Verbindung von Ring und Wendel herzustellen, kann in einer weiteren Ausführungsform der Ring nach dem Eindrehen an mehreren Stellen der ersten Wendel befestigt werden, beispielsweise mittels Laserschweißen oder durch elektrisch leitfähige selbstaushärtende Kunststoffe oder Klebstoffe.

Der Aufbau und die Zusammensetzung eines Ausführungsbeispiels eines erfindungsgemäßen Federkontaktbauteils und einer Steckerkontaktbuchse eines erfindungsgemäßen Headers werden nachfolgend anhand von Figuren erläutert. Ferner wird das erfindungsgemäße Herstellungsverfahren für die Herstellung eines erfindungsgemäßen Federkontaktbauteils anhand von Figuren erläutert. Dabei bilden alle abgebildeten und/oder beschriebenen Merkmale den Gegenstand der Erfindung, auch unabhängig von ihrer Zusammenfassung in den Ansprüchen oder deren Rückbeziehung.

Es zeigen schematisch:
- Fig. 1 bis 3: ein Ausführungsbeispiel eines erfindungsgemäßen Federkontaktbauteils in Form einer torusförmigen Kontaktfeder in drei verschiedenen perspektivischen Ansichten von der Seite,
- Fig. 4: eine Steckerkontaktbuchse eines erfindungsgemäßen Anschlussgehäuses in einer perspektivischen Ansicht von der Seite,
- Fig. 5: einen Querschnitt der Steckerkontaktbuchse gemäß Fig. 4 in einer perspektivischen Ansicht von der Seite,
- Fig. 6: den Querschnitt gemäß Fig. 5 in einer Ansicht von vorn und
- Fig. 7 bis 10: das erfindungsgemäße Verfahren zur Herstellung des erfindungsgemäßen Federkontaktbauteils in Form einer torusförmigen Kontaktfeder gemäß Fig. 1 bis 3 in mehreren Schritten.

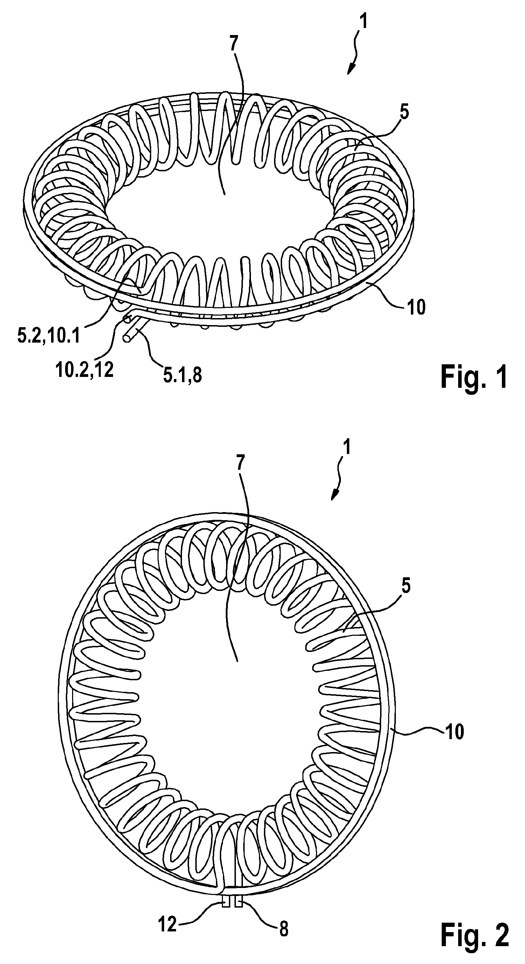

Im Folgenden wird als ein Ausführungsbeispiel ein Federkontaktbauteil in Form einer torusförmigen Kontaktfeder beschrieben. Die in den Figuren 1 bis 3 dargestellte erfindungsgemäße torusförmige Kontaktfeder 1 für eine Steckerkontaktbuchse eines elektromedizinischen Implantats weist eine ring- oder torusförmige Wendel 5 auf, welche eine durchgehende Kontaktierungsöffnung 7 mittig ausbildet. In diese durchgehende Kontaktierungsöffnung 7 kann der Stecker oder das Anschlussstück der Elektrodenleitung eingesteckt werden, wenn die Kontaktfeder 1 in einer Steckerkontaktbuchse angeordnet ist.

Die torusförmige Wendel 5 besteht bevorzugt aus einem Pt80Ir20-Draht mit einem beliebigen Querschnitt, besonders bevorzugt jedoch ein kreisförmiger, elliptischer oder rechteckförmiger Querschnitt. Die Wendel 5 umgibt den äußeren Umfang der kreis- oder ellipsenförmigen durchgehenden Steckeröffnung 7 vollständig. Das erste Ende 5.1 der Wendel 5 bildet einen ersten Anschlussstift 8 aus. Das zweite Ende der Wendel 5.2 geht übergangslos in den Ring 10 über und endet im Anschlussstift 12. Mit anderen Worten sind die Wendel 5, der Ring 10 als formstabilisierendes draht- und/oder bandförmiges Element sowie der Anschlussstift 12 einstückig ausgebildet.

In dem in den Figuren 1 bis 3 dargestellten Ausführungsbeispiel der torusförmigen Kontaktfeder 1 ist der Ring 10 als zweifacher Ring ausgebildet, der im Bereich des äußeren Umfangs der torusförmigen Wendel 5 anliegt und diese in ihrer Form stabilisiert. Der Ring 10 ist hierfür an mehreren Stellen seines Verlaufs mittels Laserschweißen oder durch selbstaushärtende elektrisch leitfähige Kunststoffe oder Klebstoffe mit der torusförmigen Wendel 5 verbunden. Das zweite Ende 10.2 des drahtförmigen Rings 10 bildet einen zweiten Anschlussstift 12 aus. Der erste Anschlussstift 8 und der zweite Anschlussstift 12 ragen in radialer Richtung von der torusförmigen Wendel 5 bzw. von dem Ring 10 nach außen vor.

In dem in den Fig. 1 bis 3 dargestellten Ausführungsbeispiel einer Kontaktfeder 1 ist diese einstückig ausgebildet, d. h. die torusförmige Wendel 5, der Ring 10 sowie die Anschlussstifte 8, 12 bestehen aus einem einzigen Stück Draht.
Gemäß der alternativ möglichen Ausführung sind die Wendel 5, der Ring 10 sowie die Anschlussstifte 8, 12 nicht einstückig ausgeführt, in Funktion jedoch identisch mit den genannten Elementen. In diesem Fall werden die einzelnen Elemente 5, 8, 10, 12 geeignet verbunden, insbesondere geschweißt oder gelötet.

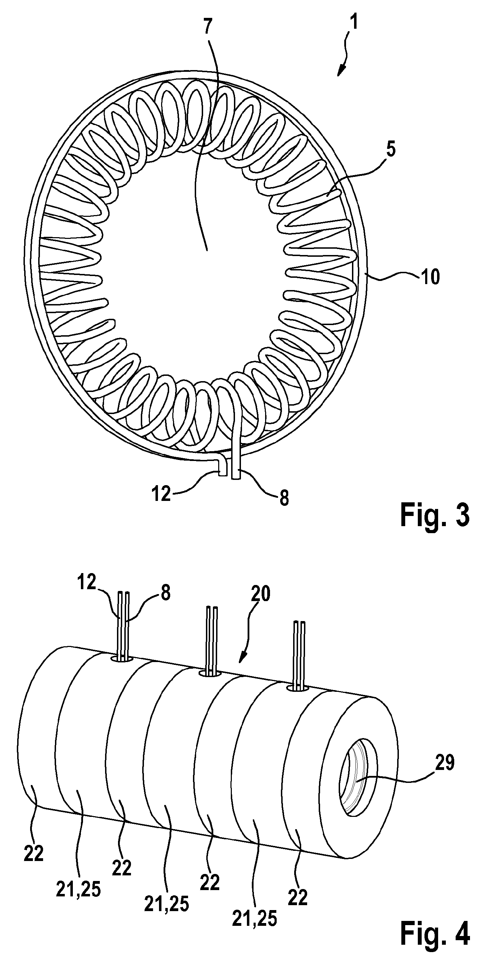

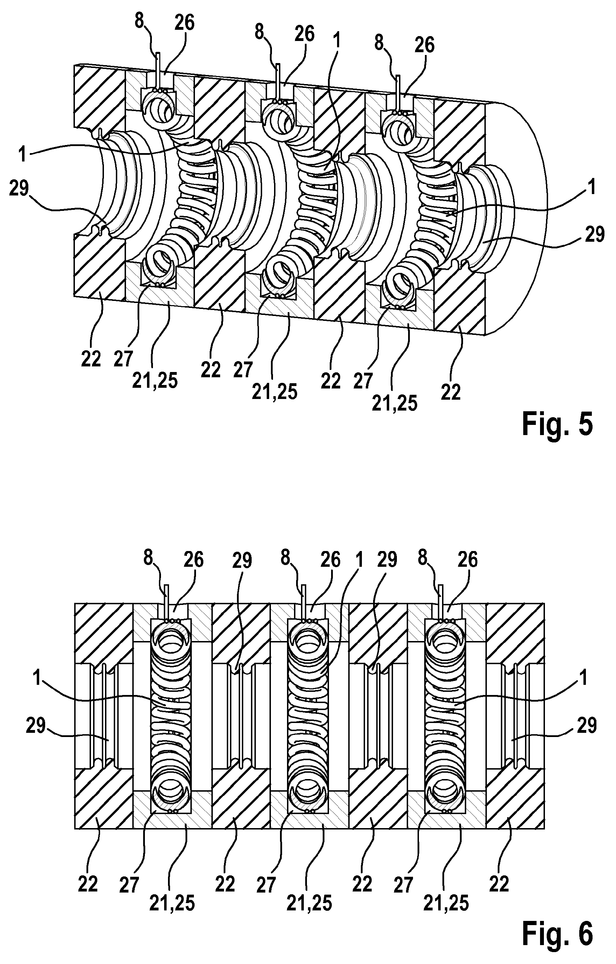

In den Figuren 4 bis 6 wird eine Steckerbuchse 20 eines erfindungsgemäßen Headers veranschaulicht, welche sich aus drei Steckerbuchsenmodulen 21 und vier Dichtlippenmodulen 22, jeweils im Wechsel zusammensetzt.
Bei beiden alternativen Lösungen kann das Gehäuse des Kontaktbuchsenbauteils aus demselben Werkstoff oder derselben Werkstoffgruppe (beispielsweise nicht leitende Polymere) bestehen, aus dem auch die Dichtbuchsenbauteile bestehen. Damit kann auch das Gehäuse des Kontaktbuchsenbauteils aus einem elektrisch isolierenden Werkstoff in Form eines kostengünstigen Spritzgussteils gefertigt werden. Der elektrisch isolierende Werkstoff kann so gewählt werden, dass die unterschiedlichen Buchsenbauteile miteinander verklebt werden können. So wird vorteilhafterweise ein mögliches Eindringen von Flüssigkeit in die elektrischen Kontaktbereich verhindert.

Jedes Kontaktbuchsenbauteil 21 weist ein beispielsweise ring- oder zylinderförmiges Gehäuse 25 mit einer mittigen, durchgehenden Öffnung auf, in dessen an der Innenwand angeordneten, innen liegende Kerbe 27 ein anhand der Figuren 1 bis 3 dargestellten Kontaktfeder 1 eingelegt ist. Jedes Gehäuse 25 ist vorzugsweise als Spritzgussteil aus Silikon gefertigt. Die am äußeren Umfang des Gehäuses 25 liegende, in radialer Richtung durchgehende Öffnung 26 dient als Durchführung für die Anschlussstifte 8, 12 der Kontaktfeder 1. Diese Öffnung 26 ist um die herausragenden Anschlussstifte (Drahtenden) 8, 12 beispielsweise mittels eines Silikonklebers abgedichtet.

Das Dichtbuchsenbauteil 22 ist ebenfalls vorzugsweise als Spritzgussteil hergestellt und umfasst ein zylindrisches Bauteil mit einer mittigen, durchgehenden Öffnung. An der innen liegenden Öffnungswand ist ein Dichtlippenpaar 29 angeordnet.

Ein in einer derartigen Steckerkontaktbuchse 20 angeordneter Stecker kann als schwimmend gelagert betrachtet werden.

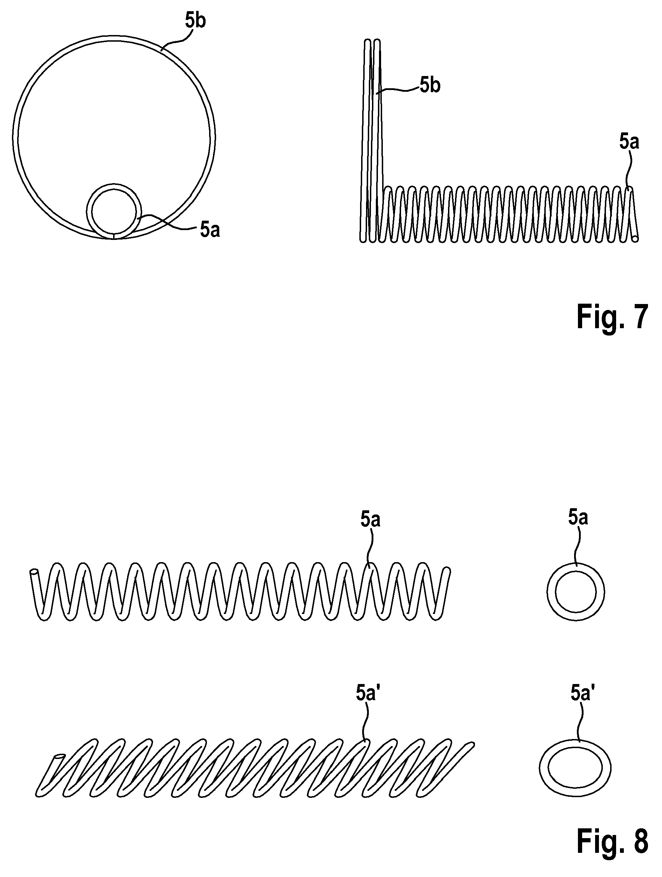

Die Herstellung des erfindungsgemäßen Federkontaktbauteils 1 wird nun anhand der Figuren 7 bis 10 beschrieben.

Das Federkontaktbauteil 1 besteht aus einem einzigen Draht, welcher beispielsweise einen kreisförmigen Querschnitt aufweist. Dieser Draht wird zunächst über einen zweistufigen Wickelprozess zu zwei ineinander übergehende, radiale Wendeln, nämlich eine erste Wendel 5a und eine zweite Wendel 5b, mit unterschiedlichen Durchmessern geformt. Die Wendeln 5a, 5b sind hierbei als radiale Wendeln ausgeführt und weisen einen einheitlichen Steigungswinkel auf (vgl. Fig. 7 mit einer Ansicht der Wendeln 5a, 5b von der Seite und einer Ansicht von vorn).

In einem zweiten Schritt wird nun die Wendel 5a mit dem kleinere Durchmesser mechanisch in einer Vorrichtung - beispielsweise in einer oder mehreren Walzen - so verformt, dass aus der radialen Wendel 5a eine geschränkte Wendel 5a' entsteht. Mit dem Grad der Schränkung werden die Federeigenschaften der Wendel 5a' in der torusförmigen Kontaktfeder vorbestimmt (siehe Fig. 8 mit jeweils einer Ansicht der Wendel 5a von vorn und von der Seite vor und nach der Schränkung). Hierbei ändert sich die äußere Form der ersten Wendel 5a mit dem kleinen Durchmesser von kreisförmig zu elliptisch.

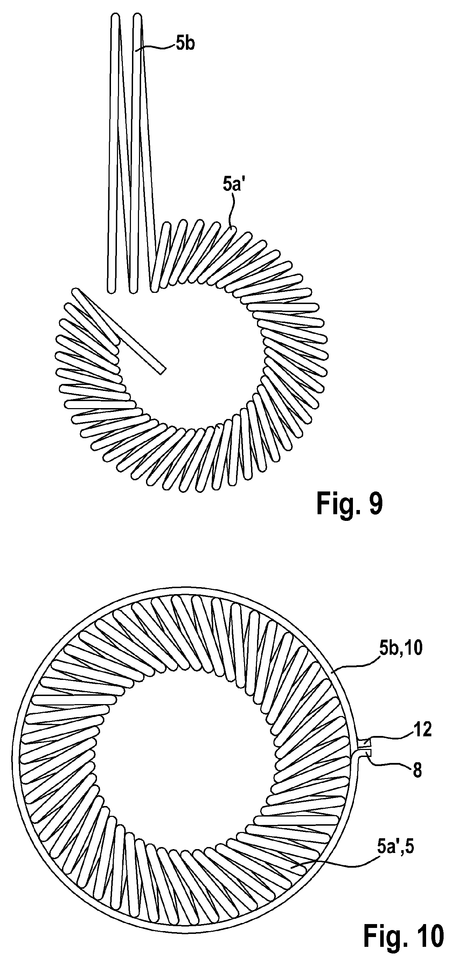

Fig. 9 zeigt den nächsten Schritt des Herstellungsverfahrens für das erfindungsgemäße Kontaktfeder in einer Ansicht von der Seite, in dem die geschränkte erste Wendel 5a' nun mechanisch in die Form eines offenen Torus gebracht wird. Anschließend wird die erste Wendel 5a', die den offenen Torus bildet, in die zweite Wendel 5b mit dem größeren Durchmesser hinein gedreht, so dass die erste Wendel 5a' nun die Wendel 5 der Darstellung gemäß Fig. 1 bis 3 und die zweite Wendel 5b mit dem größeren Durchmesser den oben beschriebenen umlaufenden Ring 10 ausbildet (siehe Fig. 10). Wie in den Figuren 1 bis 3 dargestellt, kann der Ring 10 beispielsweise zwei Windungen aufweisen, d. h. als zweifacher Ring 10 ausgebildet sein. Der Ring 10 kann alternativ aber auch lediglich eine einzige Windung oder mehr als zwei Windungen besitzen.

Der umlaufende Ring 10 wird dann mit der ersten Wendel 5a' bzw. 5 mittels Laserschweißen mit einigen Laserschweißpunkten oder durch selbstaushärtende elektrisch leitfähige Kunststoffe oder Klebstoffe verbunden. Das erste Drahtende 5.1 der Wendel 5 und das zweite Drahtende 10.2 des Rings 10 bilden jeweils einen Anschlussstift 8, 12 und werden derart verformt (gebogen), dass die Anschlussstifte 8, 12 radial von der Wendel 5, dem Ring 10 bzw. der Kontaktfeder 1 nach außen ragen. Diese Anschlussstifte 8, 12 sind in einem späteren Prozessschritt zur elektrisch leitenden Anbindung an die im Gehäuse des Implantats vorgesehenen Elektronikelemente bestimmt.

### Bezugszeichenliste

- 1: Federkontaktbauteil in Form einer torusförmigen Kontaktfeder mit einer umgebenden Wendel
- 5: Wendel
- 5.1: erstes Ende der Wendel 5
- 5.2: zweites Ende der Wendel 5
- 5a, 5a': erste Wendel
- 5b: zweite Wendel
- 7: durchgehende Kontaktierungsöffnung
- 8: Anschlussstift
- 10: Ring
- 10.1: erstes Ende des Rings 10
- 10.2: zweites Ende des Rings 10
- 12: Anschlussstift
- 20: Steckerkontaktbuchse
- 21: Kontaktbuchsenbauteil
- 22: Dichtbuchsenbauteil
- 25: Gehäuse
- 26: Öffnung
- 27: Kerbe
- 29: Dichtlippenpaar

## Patentansprüche

1. Federkontaktbauteil (1) für eine Steckkontaktbuchse eines elektromedizinischen Implantats mit einer den äußeren Umfang einer Steckeröffnung (7) umgebenden Wendel (5) mit einem ersten und einem zweiten Ende (5.1, 5.2), wobei das zweite Ende (5.2) der Wendel (5) elektrisch und mechanisch über ein draht- und/oder bandförmiges Element (10) direkt mit einem Anschlussstift (12) verbunden ist, **dadurch gekennzeichnet, dass** das draht- und/oder bandförmige Element (10) ein die Wendel (5) im Bereich ihres Umfangs außen umgebendes formstabilisierendes Mittel aufweist, das vorzugsweise als einfacher oder mehrfacher Ring oder als Spirale ausgebildet ist.

2. Federkontaktbauteil (1) nach Anspruch 1, **dadurch gekennzeichnet, dass** das zweite Ende (5.2) der Wendel (5) und das draht- und/oder bandförmige Element (10) sowie ein Anschlussstift (12) einstückig ausgebildet sind.

3. Federkontaktbauteil nach nach einem der Ansprüche 1 oder 2, **dadurch gekennzeichnet, dass** das formstabilisierende Mittel an mehreren Stellen mit der Wendel verbunden ist.

4. Federkontaktbauteil nach einem der Ansprüche 1 oder 2, **dadurch gekennzeichnet, dass** das erste Ende (5.1) der Wendel (5) einen Anschlussstift (8) ausbildet.

5. Federkontaktbauteil nach einem der Ansprüche 1 bis 4, **dadurch gekennzeichnet, dass** die Wendel (5) und das draht- und/oder bandförmige Element (10) aus dem gleichen, elektrisch leitenden Material gefertigt sind.

6. Federkontaktbauteil nach einem der Ansprüche 1 bis 5, **dadurch gekennzeichnet, dass** die Wendel (5) eine Torusfeder und das draht- und/oder bandförmige Element (10) ein(e) die Torusfeder im Bereich ihres Umfangs außen umgebende(r) einfacher oder mehrfacher Ring oder eine Spirale ist.

7. Kontaktbuchsenbauteil (21) für ein elektromedizinisches Implantat mit einem Federkontaktbauteil (1) nach den vorhergehenden Ansprüchen.

8. Kontaktbuchsenbauteil (21) nach Anspruch 7, **dadurch gekennzeichnet, dass** das Federkontaktbauteil (1) in ein Gehäuse (25) aus einem nichtleitenden Material, vorzugsweise in ein mittels Spritzguss gefertigtes Gehäuse, eingelegt oder eingebettet ist, wobei der erste Anschlussstift (8) und der zweite Anschlussstift (12) vorzugsweise im Bereich des äußeren Umfangs aus dem Gehäuse (25) herausragen.

9. Kontaktbuchsenbauteil (21) nach Anspruch 7 oder 8, **dadurch gekennzeichnet, dass** das Gehäuse (25) als Zylinderabschnitt mit mittiger, durchgehender Öffnung gestaltet ist, das im Bereich der innen liegenden Wand der Öffnung eine Kerbe (27) zur Aufnahme des Federkontaktbauteils (1) aufweist.

10. Kontaktbuchsenbauteil (21) nach einem der Ansprüche 7 bis 9, **dadurch gekennzeichnet, dass** im Gehäuse (25) mindestens eine im Wesentlichen radial verlaufende durchgehende Öffnung (26) vorgesehen ist, welche sich in einer besonders bevorzugten Ausführungsform bis in die Kerbe (27) für das Federkontaktbauteil (1) erstreckt.

11. Anschlussgehäuse für ein elektromedizinisches Implantat, **dadurch gekennzeichnet, dass** es mindestens zwei nebeneinander angeordnete Kontaktbuchsenbauteile (21) nach einem der Ansprüche 7 bis 10 aufweist.

12. Anschlussgehäuse nach Anspruch 11, **dadurch gekennzeichnet, dass** mindestens zwei Kontaktbuchsenbauteile (21) durch ein dazwischen angeordnetes Dichtbuchsenbauteil (22) getrennt ist.

13. Verfahren zur Herstellung eines Federkontaktbauteils (1) für eine Steckerkontaktbuchse eines elektromedizinischen Implantats mit einer den äußeren Umfang einer durchgehenden Kontaktierungsöffnung (7) umgebenden Wendel (5) mit den folgenden Schritten:
- Herstellung einer ersten Wendel (5a) und einer zweiten Wendel (5b) aus einem Draht oder einem Band, wobei die erste Wendel (5a) einen kleineren Durchmesser als die zweite Wendel (5b) aufweist,
- Ausbildung eines offenen Torus aus der ersten Wendel (5a) mit dem kleineren Durchmesser,
- gegebenenfalls Verbinden der ersten Wendel (5a) und der zweiten Wendel (5b) an jeweils einem Ende jeder Wendel (5a,5b) miteinander,
- Eindrehen der ersten Wendel (5a) in Form eines offenen Torus in die zweite Wendel (5b) derart, dass die zweite Wendel eine(n) die erste Wendel im Bereich ihres Umfangs außen umgebenden einfachen oder mehrfachen Ring oder Spirale (10) ausbildet.

14. Verfahren nach Anspruch 13, **dadurch gekennzeichnet, dass** die erste Wendel (5a) vor der Ausbildung des Torus geschränkt wird.

15. Verfahren nach einem der Ansprüche 13 oder 14, **dadurch gekennzeichnet, dass** der Ring (10) nach dem Eindrehen an mehreren Stellen der ersten Wendel (5a) befestigt wird, vorzugsweise mittels Laserschweißen oder durch elektrisch leitfähige selbstaushärtende Kunststoffe oder Klebstoffe.

## Claims

1. A spring contact component (1) for a plug socket of an electromedical implant with a helix (5) that surrounds the outer perimeter of a plug opening (7) and that has a first and a second end (5.1, 5.2), the second end (5.2) of the helix (5) being directly connected, electrically and mechanically, through a wire and/or band-shaped element (10), with a connection pin (12), **characterized in that** the wire and/or band-shaped element (10) has shape-stabilizing means that surround the helix (5) on the outside in the area of its perimeter and that preferably are in the form of a single or multiple ring or a spiral.

2. The spring contact component (1) described in claim 1, **characterized in that** the second end (5.2) of the helix (5) and the wire and/or band-shaped element (10) and a connection pin (12) are made as a single piece.

3. The spring contact component described in one of claims 1 or 2, **characterized in that** the shape-stabilizing means are connected with the helix in several places.

4. The spring contact component described in one of claims 1 or 2, **characterized in that** the first end (5.1) of the helix (5) forms a connection pin (8).

5. The spring contact component described in one of claims 1 through 4, **characterized in that** the helix (5) and the wire and/or band-shaped element (10) are made from the same electrically conductive material.

6. The spring contact component described in one of claims 1 through 5, **characterized in that** the helix (5) is a toroidal spring and the wire and/or band-shaped element (10) is a single or multiple ring or a spiral surrounding the toroidal spring on the outside in the area of its perimeter.

7. A jack component (21) for an electromedical implant with a spring contact component (1) described in one of the preceding claims.

8. The jack component (21) described in claim 7, **characterized in that** the spring contact component (1) is inserted or embedded into a housing (25) made of a nonconductive material, preferably into a housing made by injection molding, the first connection pin (8) and the second connection pin (12) projecting out of the housing (25), preferably in the area of the outer perimeter.

9. The jack component (21) described in claim 7 or 8, **characterized in that** the housing (25) is in the form of a cylindrical section that has an axial through opening and that has, in the area of the inner wall of the opening, a notch (27) to hold the spring contact component (1).

10. The jack component (21) described in one of claims 7 through 9, **characterized in that** the housing (25) has at least one essentially radial through opening (26) that in an especially preferred embodiment extends all the way into the notch (27) for the spring contact component (1) .

11. A connector housing for an electromedical implant, **characterized in that** it has at least two jack components (21) as described in one of claims 7 through 10 that are arranged next to one another.

12. The connector housing described in claim 11, **characterized in that** at least two jack components (21) [are] separated by a sealing jack component (22) arranged between them.

13. A process for producing a spring contact component (1) for a plug contact of an electromedical implant with a helix (5) surrounding the outer perimeter of a through contacting opening (7), the process comprising the following steps:
- Producing a first helix (5a) and a second helix (5b) from a wire or a band, the first helix (5a) having a smaller diameter than the second helix (5b);
- Forming an open torus from the first helix (5a) with the smaller diameter;
- Possibly connecting the first helix (5a) and the second helix (5b) together at one end of each helix (5a, 5b);
- Twisting the first helix (5a) in the form of an open torus inside of the second helix (5b) in such a way that the second helix forms a single or multiple ring or spiral (10) that surrounds the first helix on the outside in the area of its perimeter.

14. The process described in claim 13, **characterized in that** the first helix (5a) is twisted before the torus is formed.

15. The process described in one of claims 13 or 14, **characterized in that** the ring (10) is fastened to several places of the first helix (5a) after the first helix (5a) is twisted inside the ring (10), this fastening preferably being done by means of laser beam welding or electrically conductive self-hardening plastics or adhesives;

## Revendications

1. Elément de contact à ressort (1) pour une prise de contact enfichable d'un implant médical, avec une bobine (5) entourant le périmètre extérieur d'une ouverture de prise (7) avec une première et une seconde extrémités (5.1, 5.2), où la seconde extrémité (5.2) de la bobine (5) est reliée électriquement et mécaniquement de façon directe avec une broche de connexion (12) par l'intermédiaire d'un élément en forme de fil et/ou de bande (10), **caractérisé en ce que** l'élément en forme de fil et/ou de bande (10) présente un moyen, qui est de préférence conçu sous la forme d'un anneau simple ou multiple ou d'une spirale, stabilisant la forme de la bobine (5) en l'entourant par l'extérieur dans la partie de son périmètre.

2. Elément de contact à ressort (1) selon la revendication 1, **caractérisé en ce que** la seconde extrémité (5.2) de la bobine (5) et l'élément en forme de fil et/ou de bande (10), ainsi que la broche de connexion (12) sont conçus en une seule pièce.

3. Elément de contact à ressort (1) selon l'une des revendications 1 ou 2, **caractérisé en ce que** le moyen stabilisant la forme est relié à plusieurs endroits avec la bobine.

4. Elément de contact à ressort (1) selon l'une des revendications 1 ou 2, **caractérisé en ce que** la première extrémité (5.1) de la bobine (5) forme une broche de connexion (8).

5. Elément de contact à ressort (1) selon l'une des revendications 1 à 4, **caractérisé en ce que** la bobine (5) et l'élément en forme de fil et/ou de bande (10) sont fabriqués dans le même matériau électriquement conducteur.

6. Elément de contact à ressort (1) selon l'une des revendications 1 à 5, **caractérisé en ce que la** bobine (5) est un ressort torique et l'élément en forme de fil et/ou de bande (10) est un anneau simple ou multiple ou une spirale entourant le ressort torique par l'extérieur dans la partie de son périmètre.

7. Elément de prise de contact (21) pour un implant médical avec un élément de contact à ressort (1) selon l'une des revendications précédentes.

8. Elément de prise de contact (21) selon la revendication 7, **caractérisé en ce que** l'élément de contact à ressort (1) est incorporé ou enseveli dans un boitier (25) à base d'un matériau non conducteur, de préférence, dans un boitier fabriqué au moyen d'un moulage par injection, où la première broche de connexion (8) et la seconde broche de connexion (12) dépassent du boitier (25) de préférence dans la partie du périmètre extérieur.

9. Elément de prise de contact (21) selon les revendications 7 ou 8, **caractérisé en ce que** le boitier (25) est conçu sous la forme d'une partie de cylindre, avec une ouverture centrale, traversant de part en part qui présente une entaille (27) dans la partie de la paroi de l'ouverture se situant à l'intérieur pour l'admission de l'élément de contact à ressort (1).

10. Elément de prise de contact (21) selon les revendications 7 à 9, **caractérisé en ce que,** dans un mode de réalisation particulièrement préféré, au moins une ouverture (26) essentiellement radiale traversant de part en part est prévue dans le boitier (25), laquelle s'étend jusqu'à l'entaille (27) pour l'élément de contact à ressort (1).

11. Boitier de connexion pour un implant électro médical, **caractérisé en ce qu**'il présente au moins deux éléments de prise de contact (21) selon l'une des revendications 7 à 10, disposés l'un à côté de l'autre.

12. Boitier de connexion selon la revendication 11, **caractérisé en ce qu**'au moins deux éléments de prise de contact (21) sont séparés par un élément de prise étanche (22) disposé entre eux.

13. Procédé de fabrication d'un élément de contact à ressort (1) pour une prise de contact enfichable d'un implant électro-médical, avec une bobine (5) entourant le périmètre extérieur d'une ouverture de contact (7) traversant de part en part, avec les étapes suivantes:
- fabrication d'une première bobine (5a) et d'une seconde bobine (5b) à base d'un fil ou d'une bande, où la première bobine (5a) présente un diamètre inférieur à celui de la seconde bobine (5b),
- formation d'un tore ouvert à partir de la première bobine (5a) avec le plus petit diamètre,
- éventuellement, connexion de la première bobine (5a) et la seconde bobine (5b) l'une à l'autre chaque fois à une extrémité de chaque bobine (5a, 5b),
- enroulement de la première bobine (5a) sous la forme d'un tore ouvert dans la seconde bobine (5b) de telle manière que la seconde bobine forme un anneau simple ou multiple ou une spirale (10) entourant par l'extérieur la première bobine dans la partie de son périmètre.

14. Procédé selon la revendication 13, **caractérisé en ce que** la première bobine (5a) est avoyée avant la formation du tore.

15. Procédé selon l'une des revendications 13 ou 14, **caractérisé en ce que** l'anneau (10) est fixé à plusieurs endroits de la première bobine (5a) après l'enroulement, de préférence au moyen d'un soudage laser ou par des matières synthétiques ou matières adhésives électriquement conductrices auto-réticulant.
